# EUROPEAN PATENT SPECIFICATION

(11) **EP 4 019 412 B1**
(45) Date of publication and mention of the grant of the patent: **12.02.2025**
(21) Application number: 21216353.9
(22) Date of filing: 21.12.2021
(51) Int. Cl.: B64F 5/30, A61L 2/00, A61L 2/10, A61L 2/08, B64D 11/06, B64D 11/00

(54) **AIRCRAFT DISINFECTION SYSTEM FOR STOWABLE ITEMS**
FLUGZEUGDESINFIZIERUNGSSYSTEM FÜR VERSTAUBARE ARTIKEL
SYSTÈME DE DÉSINFECTION D'AÉRONEF POUR ARTICLES ESCAMOTABLES

(30) Priority: 28.12.2020 US 202017134981
(43) Date of publication of application: 29.06.2022
(73) Proprietor: Goodrich Corporation, Charlotte, NC 28217-4578 (US)
(72) Inventor: DOWTY, Mark B., Rural Hall, 27045 (US); HUSMANN, Ethan, Fairfax, VA, 22032 (US); ST. ROCK, Brian, Andover, 06232 (US)
(74) Representative: Dehns

(56) References cited:
- EP-A2- 3 925 631
- EP-B1- 3 293 118
- DE-U1- 202020 104 890
- US-A1- 2016 250 362
- US-A1- 2017 107 659
- US-A1- 2019 060 496

## Description

### FIELD

The present disclosure relates to devices, systems, and methods for pathogen disinfection, and in particular to disinfection systems for stowable items in an aircraft cabin.

### BACKGROUND

The recent novel-coronavirus (SARS-COV-2) outbreak has negatively impacted the safety and health of many people. Pathogens can be transmitted via direct airborne transmission between users or via indirect contact transmission from different users occupying the same space at different times. For example, pathogens on personal items of passengers or lingering pathogens that may remain on contact surfaces of an aircraft cabin between flights may spread to passengers and/or crew members. The safety of passengers and crew members may be improved by performing disinfecting treatments to surfaces, such as seats, ceiling/wall panels, handles, and lavatory surfaces, etc., to mitigate the presence of pathogens on such surfaces. However, conventional disinfection procedures may inadequately sanitize certain stowable items or hidden areas within a cabin of an aircraft. Accordingly, extra cleaning between flights may be required to disinfect stowable items or areas of an aircraft cabin, and thus the operating efficiency of the aircraft (increased interval time between flights) may be adversely affected. Further, the effectiveness and quality of such conventional treatments are often difficult to verify/track. Still further, certain treatments, such as ultraviolet radiation treatments, may result in harm to the operator if not properly effectuated. US 2016/250362 A1 discloses a system for sanitizing a tray table including a sanitation assembly operatively coupled to the tray table. The sanitation assembly includes an ultraviolet (UV) light source configured to emit UV light, and a sanitation control unit operatively coupled to the UV light source. The sanitation control unit operates the UV light source to emit the UV light onto the tray table when the tray table is secured in an upright position, and prevents the UV light source from emitting the UV light when the tray table is not secured in the upright position. EP 3,293,118 B1 and US 2019/060496 A1 relate to UV light sanitizing systems and methods. US 2017/107659 A1 relates to systems and methods for refreshing passenger garments on board a vehicle.

### SUMMARY

In various embodiments, the present disclosure provides a disinfection system for a cabin of an aircraft according to claim 1.

The disinfecting electromagnetic radiation that is configured to be emitted from the electromagnetic radiation source may be ultraviolet ("UV") radiation and may comprise a wavelength between about 10 nanometers and about 405 nanometers. In various embodiments, the disinfecting electromagnetic radiation configured to be emitted from the electromagnetic radiation source is far UV-C radiation and comprises a wavelength between about 100 nanometers and about 280 nanometers. In various embodiments, the disinfecting electromagnetic radiation configured to be emitted from the electromagnetic radiation source is UV-B radiation and comprises a wavelength between about 280 nanometers and about 315 nanometers. In various embodiments, the disinfecting electromagnetic radiation configured to be emitted from the electromagnetic radiation source is UV-A radiation and comprises a wavelength between about 315 nanometers and about 405 nanometers. In various embodiments, the housing comprises at least one of a reflective material, a refractive material, and a diffractive material configured to direct emission of the disinfecting electromagnetic radiation.

The disinfection system further includes a controller electrically coupled to the electromagnetic radiation source. The controller comprises a processor and a tangible, non-transitory computer-readable storage medium having instructions stored thereon that, in response to execution by the processor, cause the processor to perform various operations. The various operations include controlling, by the processor, emission of the disinfecting electromagnetic radiation. The disinfection system includes a detector coupled to the housing and electrically coupled to the controller. The detector is configured to determine, by the processor, at least one of a presence, a position, and an orientation of the stowable item. Controlling the emission of the disinfecting electromagnetic radiation is based on at least one of the presence, the position, and the orientation of the stowable item. In various embodiments, controlling the emission of the disinfecting electromagnetic radiation comprises varying the wavelength of the disinfecting electromagnetic radiation. In various embodiments, the controller is configured to determine, by the processor, a dosage of the disinfecting electromagnetic radiation delivered to the stowable item.

In various embodiments, the stowable item is at least one of a personal item of a passenger and a component of the cabin of the aircraft. For example, the housing may include a passenger storage container and the stowable item may be a personal item of a passenger. Not within the scope of the claims, however mentioned as an example, a passenger storage container comprises a lid, wherein controlling the emission of the disinfecting electromagnetic radiation is based on whether the lid is open or closed. In various embodiments, the housing comprises seat structure, the stowable item comprises a tray, and the compartment is a receptacle for at least partially receiving the tray. The seat structure may be a seat-back and the electromagnetic radiation source may be mounted to the seat-back. For example, the electromagnetic radiation source may be disposed along a shoulder of the seat-back that defines the receptacle. In various embodiments, the seat structure is an armrest and the electromagnetic radiation source is disposed within the receptacle. The housing comprises a refractive material configured to direct emission of the disinfecting electromagnetic radiation.

Also disclosed herein, according to various embodiments, is a method of delivering disinfecting electromagnetic radiation to a cabin of an aircraft according to claim 13.

The forgoing features and elements may be combined in various combinations without exclusivity, unless expressly indicated herein otherwise. These features and elements as well as the operation of the disclosed embodiments will become more apparent in light of the following description and accompanying drawings.

### BRIEF DESCRIPTION OF THE DRAWINGS

FIG. 1A illustrates a view of a cabin of an aircraft, in accordance with various embodiments;
FIGS. 1B, 1C, and 1D are schematic block diagrams of disinfection systems, in accordance with various embodiments;
FIG. 2 is a perspective view of a passenger area of an aircraft cabin, in accordance with various embodiments;
FIG. 3 is a perspective view of a disinfection system implemented in conjunction with a personal item storage compartment, in accordance with various embodiments;
FIG. 4 is a perspective view of a disinfection system implemented in conjunction with a seat-back tray, in accordance with various embodiments;
FIGS. 5, 6A, and 6B are perspective top views of a disinfection system implemented in conjunction with an armrest tray storage compartment, in accordance with various embodiments;
FIG. 7 is a perspective view of a disinfection system implemented in conjunction with a magazine or brochure storage sleeve/pocket, in accordance with various embodiments; and
FIG. 8 is a schematic flow chart diagram of a method of delivering disinfecting electromagnetic radiation to a cabin of an aircraft

The subject matter of the present disclosure is particularly pointed out and distinctly claimed in the concluding portion of the specification. A more complete understanding of the present disclosure, however, may best be obtained by referring to the detailed description and claims when considered in connection with the drawing figures.

### DETAILED DESCRIPTION

The detailed description of exemplary embodiments herein makes reference to the accompanying drawings, which show exemplary embodiments by way of illustration. While these exemplary embodiments are described in sufficient detail to enable those skilled in the art to practice the disclosure, it should be understood that other embodiments may be realized and that logical changes and adaptations in design and construction may be made in accordance with this disclosure and the teachings herein without departing from the scope of the disclosure defined by the appended claims. Thus, the detailed description herein is presented for purposes of illustration only and not of limitation.

Disclosed herein, according to various embodiments, are devices, systems, methods, and articles of manufacture for delivering disinfecting electromagnetic radiation to various areas and components of a cabin of an aircraft. For example, the present disclosure generally provides a system that may be configured to emit electromagnetic radiation to disinfect a stowable item, according to various embodiments. Generally, the devices, systems, methods, and articles of manufacture disclosed and described herein facilitate disinfection treatments, especially to items and/or components that would not be subjected to conventional aircraft cleaning treatments. Although numerous details and examples are included herein pertaining to utilizing these concepts to aircraft cabins, the present disclosure is not necessarily so limited, and thus aspects of the disclosed embodiments may be adapted for performance in a variety of other industries (e.g., trains, vehicles, buildings, hotels, etc.). As such, numerous applications of the present disclosure may be realized.

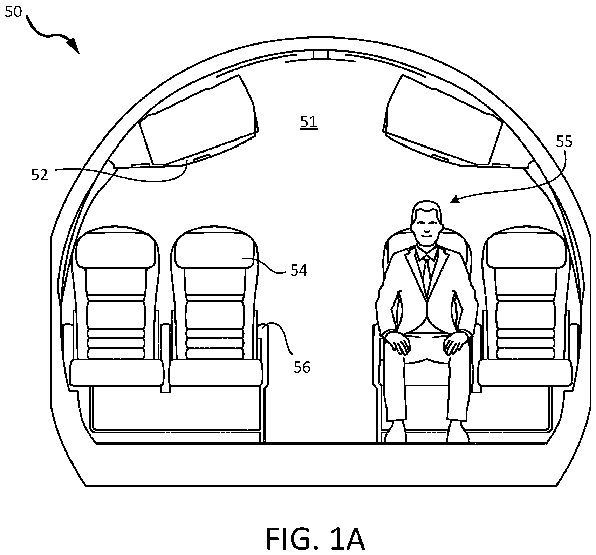

With reference to FIG. 1A, a cabin 51 of an aircraft 50 is shown, according to various embodiments. The aircraft 50 may be any aircraft such as an airplane, a helicopter, or any other aircraft. Pathogens, such as viruses and bacteria, may remain on surfaces of the cabin 51, and these remaining pathogens may result in indirect contact transmission to other people (e.g., subsequent passengers). For example, the cabin 51 may include overhead bins 52, passenger seats 54 for supporting passengers 55, armrests 56, lavatory surfaces, and other structures/surfaces upon which active pathogens may temporarily reside. As mentioned above, certain components within an aircraft cabin, such as stowable items, may not receive adequate conventional disinfection treatment. Thus, the present disclosure provides a disinfection system that is configured to deliver disinfecting electromagnetic radiation to stowable items, such as personal items of a passenger (e.g., a cell phone or wallet) or retractable/foldable component of the aircraft cabin, such as a tray or a section of a seat.

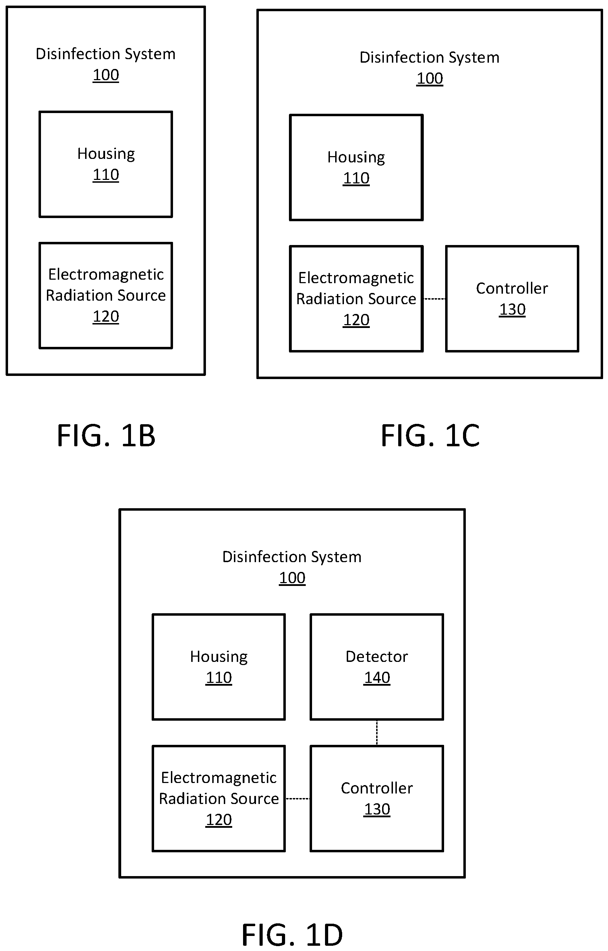

In various embodiments, and with reference to FIG. 1B, the disinfection system 100 includes among other things, a housing 110 and an electromagnetic radiation source 120. The housing 110 generally defines a compartment within which a stowable item is configured to be at least partially retained, according to various embodiments. The electromagnetic radiation source 120 may be coupled to the housing 110 and may be configured to operably emit disinfecting electromagnetic radiation to the compartment to deliver a disinfecting treatment to the stowable item. **In** various embodiments, the stowable item may have a stowed position and a released/extended position. As described in greater detail below, the electromagnetic radiation source 120 may be configured to deliver the disinfecting treatment to the stowable item when it is in the stowed position, thus potentially enabling the disinfecting electromagnetic radiation to remain in the localized vicinity of the stowed stowable item (e.g., within the compartment). **In** such configurations, the disinfecting electromagnetic radiation may be prevented from making inadvertent contact with passengers or crew of the aircraft.

As used herein, the term "electromagnetic radiation source" 120 refers to a lighting unit or other device that is configured to selectively emit radiation that is at least partially effective at inactivating and/or inhibiting pathogens. As used herein, "pathogens" may refer to bacteria, viruses, fungal spores, and other microorganisms that may cause disease in mammals. **In** various embodiments, the electromagnetic radiation source 120 emits ultraviolet ("UV") radiation. That is, the term "disinfecting electromagnetic radiation" may refer to UV radiation. In various embodiments, the electromagnetic radiation source 120 is a light-emitting diode ("LED") configured to emit UV radiation. The electromagnetic radiation source 120 may be referred to as a lighting unit and may include a plurality of discrete LEDs (e.g., an array of LEDs) that are controlled to collectively produce a desired intensity/wavelength of UV radiation. The lighting unit may include associated circuitry coupled to a controller, as described in greater detail below, for controlling emission of the UV radiation.

In various embodiments, the disinfecting electromagnetic radiation configured to be emitted from the electromagnetic radiation source comprises a wavelength between about 10 nanometers and about 405 nanometers. In various embodiments, the disinfecting electromagnetic radiation configured to be emitted from the electromagnetic radiation source is referred to as "far UV radiation" or "UV-C radiation" and comprises a wavelength between about 100 nanometers and about 280 nanometers. For example, the disinfecting electromagnetic radiation may have a wavelength of between about 220 nanometers and about 260 nanometers. In various embodiments, the disinfecting electromagnetic radiation configured to be emitted from the electromagnetic radiation source is referred to as "hard UV radiation" or "UV-B radiation" and comprises a wavelength between about 280 nanometers and about 315 nanometers. In various embodiments, the disinfecting electromagnetic radiation configured to be emitted from the electromagnetic radiation source is referred to as "near UV radiation" or "UV-A radiation" and comprises a wavelength between about 315 nanometers and about 405 nanometers. As used in this context only, the term "about" refers to plus or minus 5 nm.

The housing 110 comprises a refractive material and may further comprise a reflective or a diffractive material configured to direct emission of the disinfecting electromagnetic radiation. That is, the surfaces and/or walls of the housing 110 that define the compartment within which the stowable item may be stowed may be specifically configured to provide desired radiation propagation properties to promote disinfection across the entire surface of the stowable item, or at least across a majority portion of the hidden regions of the stowable item.

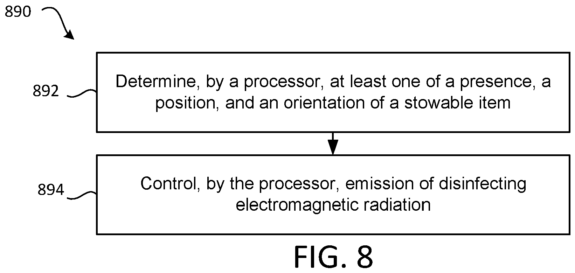

With reference to FIG. 1C, the disinfection system 100 further includes a controller 130 electrically coupled to the electromagnetic radiation source. The controller 130 comprises a processor and a tangible, non-transitory computer-readable storage medium having instructions stored thereon that, in response to execution by the processor, cause the processor to perform various operations. As described in greater detail below, the various operations performed by the processor include controlling, by the processor, emission of the disinfecting electromagnetic radiation. Controlling or actuating emission of the UV radiation may be performed in response to various conditions or in conjunction with other aircraft systems, such as dynamic power management systems of an aircraft seat. Additional details pertaining to controller operations are provided below with reference to FIGS. 1D and 8.

In various embodiments, the controller 130 may be configured to be electrically coupled to the circuitry (e.g., integrated circuit components) of the electromagnetic radiation source 120. The controller 130 may be affixed/integrated into the circuitry of the lighting unit or the controller 130 may be integrated into computer systems onboard the aircraft. The controller 130 comprises a processor. In various embodiments, the controller 130 is implemented in a single processor. In various embodiments, the controller 130 may be implemented as and may include one or more processors and/or one or more tangible, non-transitory memories and be capable of implementing logic. Each processor can be a general purpose processor, a digital signal processor (DSP), an application specific integrated circuit (ASIC), a field programmable gate array (FPGA) or other programmable logic device, discrete gate or transistor logic, discrete hardware components, or any combination thereof. The controller 130 may comprise a processor configured to implement various logical operations in response to execution of instructions, for example, instructions stored on a non-transitory, tangible, computer-readable medium (i.e., the memory) configured to communicate with the controller 130. Furthermore, any number of conventional techniques for electronics configuration, signal processing and/or control, data processing and the like may be employed. Also, the processes, functions, and instructions may can include software routines in conjunction with processors, etc.

System program instructions and/or controller instructions may be loaded onto a non-transitory, tangible computer-readable medium having instructions stored thereon that, in response to execution by the processor, cause the controller to perform various operations. The term "non-transitory" is to be understood to remove only propagating transitory signals per se from the claim scope and does not relinquish rights to all standard computer-readable media that are not only propagating transitory signals per se.

The instructions stored on the memory of the controller 130 may be configured to perform various operations. The schematic flow chart diagram of FIG. 8 include various exemplary controller methods that the processor of the controller 130 may perform. Generally, the controller 130 is configured to control, by the processor, the actuation and intensity of UV radiation emitted from the respective LEDs that form the electromagnetic radiation source.

With reference to FIG. 1D, the disinfection system 100 also includes a detector 140 coupled to the housing 110 and electrically coupled to the controller 130. The detector 140 is configured to determine, by the processor of the controller 130, at least one of a presence, a position, and an orientation of the stowable item. For example, the detector 140 may be generally configured to determine if the stowable item is in the stowed position (i.e., if the stowable item is properly positioned within the compartment defined by the housing 110). In response to the detector 140 and the controller 130 determining that the stowable item is in a desired position relative to the housing 110, the controller 130 may actuate the electromagnetic radiation source 120 to deliver disinfecting treatment to the stowable item. Said differently, controlling the emission of the disinfecting electromagnetic radiation is based on at least one of the presence, the position, and the orientation of the stowable item. For example, controlling the emission of the disinfecting electromagnetic radiation may include changing, altering, and/or varying the wavelength of the disinfecting electromagnetic radiation.

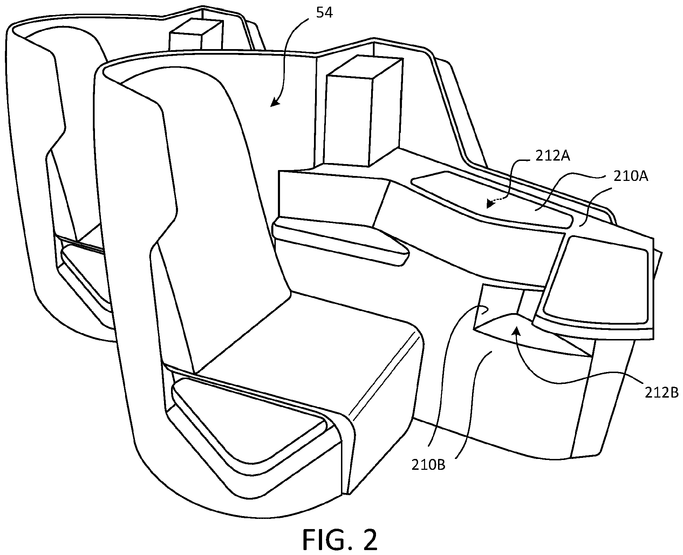

In various embodiments, the controller 130 is also configured to determine, by the processor, a dosage of the disinfecting electromagnetic radiation delivered to the stowable item. In response to determining that a sufficient dosage of the disinfecting treatment has been delivered to the stowable item, the electromagnetic radiation source may be adjusted to turn off or at least lower the intensity of the UV radiation, thereby conserving power. Numerous examples of the disinfection system 100 incorporated into various regions and structures of the cabin of the aircraft are provided below with reference to FIGS. 2, 3, 4, 5, 6A, 6B, and 7. In said figures, the controller, the detector, and even the electromagnetic light source may not be visible (e.g., may be hidden from view). Said differently, the controller and/or the detector may be integrated within the housing or may otherwise be disposed away from the housing in a relatively remote location.

In various embodiments, and with reference to FIG. 2, a passenger region of an aircraft cabin may include a seat 54, a tray, a shelf, and various compartments for storing personal items of the passenger. In various embodiments, the passenger region may also include a compartment where integrated passenger controls are housed. These compartments, such as compartment 212A and 212B, may be defined by housing 210A and 210B, respectively. Thus, the term "housing" as used in this context may refer to aircraft cabin structure in proximity to passengers or crew of the aircraft. In various embodiments, an electromagnetic radiation source may be mounted within the one or more compartments 212A, 212B to provide disinfecting electromagnetic radiation to the personal items placed by the passenger within the defined compartments. For example, a passenger may insert keys, wallet, a phone, or other personal items into one or more of the storage compartments, and these items may be the stowable items that are configured to be sanitized by the UV radiation emitted from the electromagnetic radiation sources mounted within the compartments.

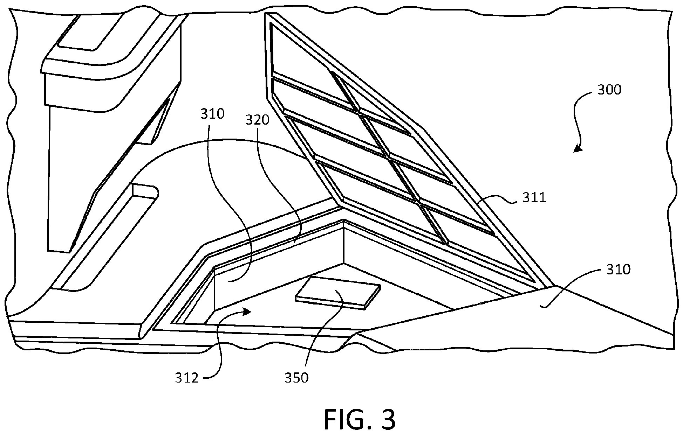

In various embodiments, and with reference to FIG. 3, a more detailed view of an exemplary storage compartment in a passenger region of an aircraft is provided. In FIG. 3, the disinfection system 300 includes housing 310 that is a shelf structure of an aircraft cabin. Defined within the housing 310 is a compartment 312 within which a stowable item 350 (e.g., a personal item of the passenger) may be deposited. The disinfection system 300 may further include one or more electromagnetic radiation sources, such as LED strip 320 mounted along sidewalls of the compartment 312. In various embodiments, the housing 310 also includes a lid 311 configured to be opened and closed by the passenger. In various embodiments, the lid 311 may be biased (e.g., via gravity or via mechanism) to be in the closed position. With the lid 311 in the closed/shut position, the electromagnetic radiation source may be triggered to provide the disinfecting treatment to the compartment 312 and the items 350 stored therein. That is, controlling, by the processor of the controller, emission of the disinfecting electromagnetic radiation may be based on whether the lid is open or closed.

In various embodiments, an item detection subsystem may be incorporated into the housing 310 or compartment 312. That is, the detector may be an item detection subsystem that is configured to determine a presence of one or more personal items 350 of the passenger in the compartment 312. In such configurations, the disinfecting treatment from the electromagnetic radiation source may be performed only when a stowable item is determined to be within the defined compartment.

Although the LED strip 320 is shown along the sidewalls of the compartment, the electromagnetic radiation source may be disposed in other configurations. For example, the bottom surface of the lid 311 may have one or more UV sources that are configured to be activated once the lid 311 is closed. In various embodiments, the intensity (e.g., wavelength) of the electromagnetic radiation is altered in response to the position of the lid 311. For example, when the lid 311 is open the electromagnetic radiation source may emit near UV radiation but when the lid 311 is closed the electromagnetic radiation source may emit far UV radiation, according to various embodiments.

As mentioned above, the housing 310 may be made from various materials that configured to refract, reflect, and/or diffract the UV radiation, thus helping to improve uniformity and consistency of the disinfecting treatment. For example, the floor of the compartment 312 may be made from a material that is configured to propagate light to the surface of the personal item 350 that is resting against the floor of the compartment 312, thus facilitating sanitization of all the surfaces of the personal item, according to various embodiments.

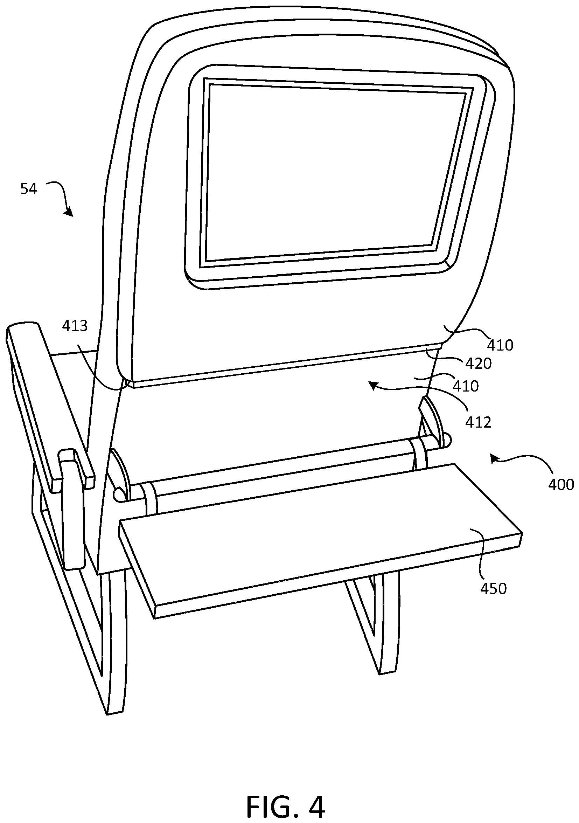

In various embodiments, and with reference to FIG. 4, the disinfection system 400 has a housing that is a seat structure 410 defining a receptacle 412 (e.g., a recess) that is configured to receive a tray 450. That is, the stowable item may be tray 450. The disinfection system 400 may include an electromagnetic radiation source 420 coupled to the seat structure 410 so as to be capable of delivering UV radiation to the tray 450 in the stowed position within the receptacle 412. For example, the seat structure 410 may be a seat-back and the electromagnetic radiation source 420 may be mounted to the seat-back. In various embodiments, the electromagnetic radiation source 420 may be disposed along a shoulder 413 of the seat-back that defines the receptacle. In various embodiments, regions of the seat-back structure may be made from a reflective, refractive, and/or diffractive material configured to propagate UV radiation to the surface of the tray 450 that faces the seat-back in the stowed position.

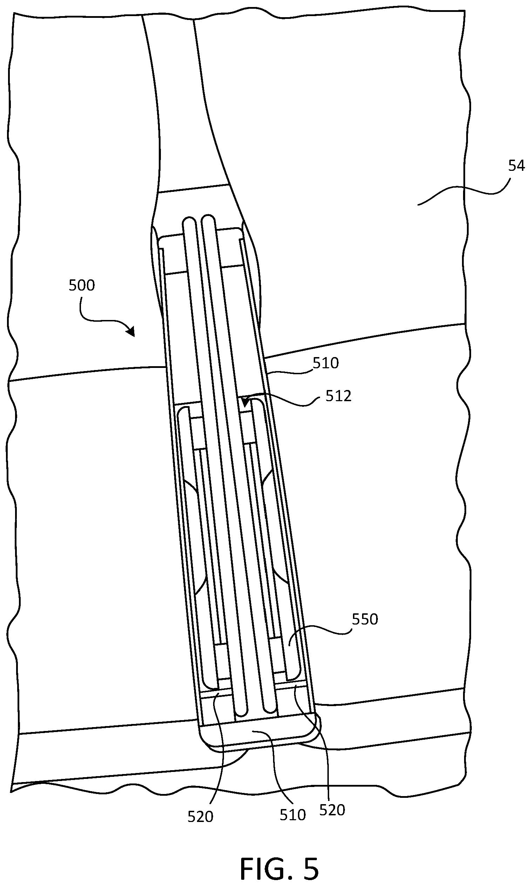

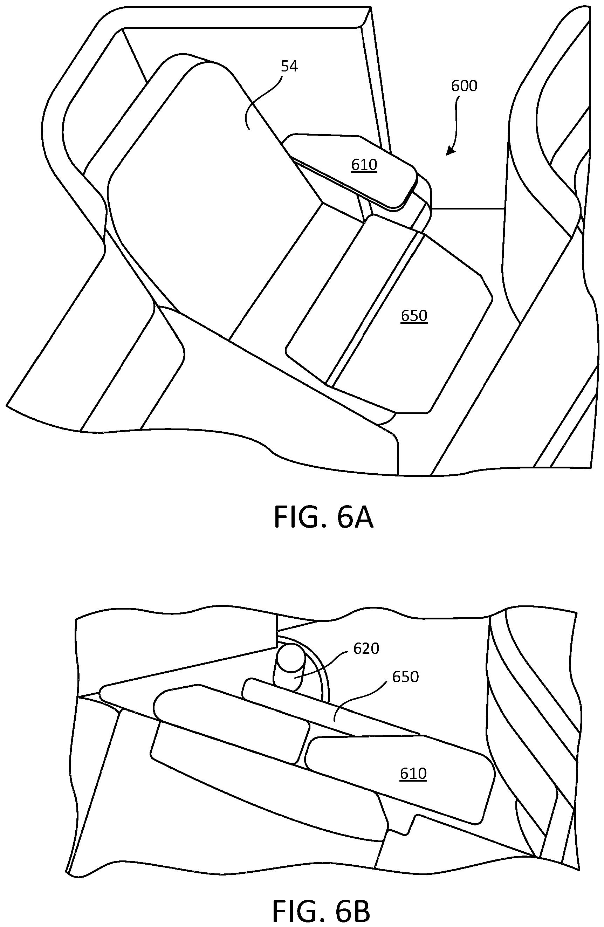

In various embodiments, and with reference to FIGS. 5, 6A, and 6B, the disinfection system 500, 600 includes a housing that is an armrest 510, 610 and the stowable item is a tray 550, 550 that is configured to be stowed within a compartment 512 defined within or adjacent to the armrest 510, 610. The disinfection system 500, 600 may also include an electromagnetic radiation source 520, 620 that is coupled to the armrest 510, 610 and/or that is disposed within the compartment 512 so as to be able to provide disinfecting UV treatment to the stowed tray 550, 650. For example, in FIG. 5 the electromagnetic radiation source 520 may disposed within and extend vertically along the armrest compartment 512 in order to deliver disinfecting treatment to the tray 550. Similar to the above, one or more surfaces/walls of the armrest 510 may be made from materials that are configured to reflect, refract, and/or diffract the UV radiation to facilitate uniform sanitization of the entire tray 550.

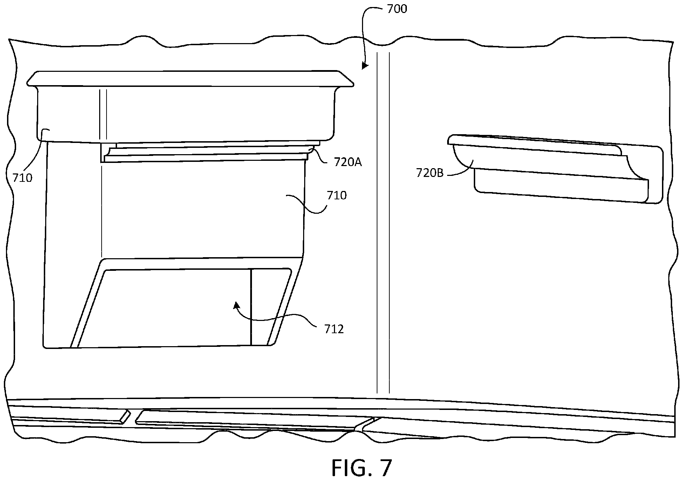

In various embodiments, and with reference to FIG. 7, the disinfection system 700 may include a housing 710 that is a magazine pocket or brochure sleeve mounted to a wall or seat of the aircraft cabin area. The housing 710 may define a compartment 712 within which magazines, brochures, or other documents (e.g., the stowable items) may be retained. The electromagnetic radiation source may be disposed within the pocket/sleeve and may thus be configured to provide disinfecting treatment to the items retained there within. In various embodiments, the disinfection system 700 may further include one or more additional electromagnetic radiation sources 720A, 720B that are outwardly facing (relative to compartment 712) and may be configured to selectively deliver UV radiation to specific/targeted areas of the passenger area of the cabin of the aircraft.

In various embodiments, and with reference to FIG. 8, a method 890 of delivering disinfecting electromagnetic radiation to a cabin of an aircraft is provided. The method 890, which may be performed by controller 130 described above, includes determining, by a processor, at least one of a presence, a position, and an orientation of a stowable item at step 892. The method 890 further includes controlling, by the processor, the emission of the disinfecting electromagnetic radiation at step 894. Step 894 may include modulating the relative intensity outputs of the one or more electromagnetic radiation sources. The method 890 includes controlling/modulating the electromagnetic radiation in response to the determined presence, position, and/or orientation of the stowable item. In various embodiments, the method 890 further includes (or step 894 may include) managing, by the processor, electric power of the seat and/or the aircraft cabin structure that is dedicated to delivering disinfecting electromagnetic radiation. For example, the controller/processor may be configured to manage power consumption and thus may be configured to selectively actuate emission of the disinfecting electromagnetic radiation when sufficient power can be dedicated to operate the disinfecting system.

In various embodiments, the method 890 also includes determining a dosage of disinfecting electromagnetic radiation delivered to the stowable item. For example, the controller may be configured to calculate a disinfection rating of an environment where the stowable item is situated. The disinfection rating may be based on at least one of an intensity of the disinfecting electromagnetic radiation, an activated time of the electromagnetic radiation source (e.g., to account for how long the disinfecting radiation has been irradiated at target surfaces of the stowable item and/or to account for luminance degradation over time), and a distance between the electromagnetic light source and the stowable item. The term "disinfection rating" may refer to a planned disinfection procedure (i.e., an estimated quantification of the extent of a disinfection treatment that will be carried out) or may refer to a performed disinfection procedure (i.e., an estimated quantification of the cleanliness of the stowable item after a disinfection treatment has been performed).

In various embodiments, the operations performed by the controller 130 include recommending supplementary disinfection procedures. That is, if the electromagnetic radiation source was not activated for a sufficient time period, the controller may ask for supplemental/extra disinfection to meet cleanliness thresholds. Accordingly, the controller 130 may communicate an alert or other notification to an aircraft crew member or other maintenance operator.

Benefits, other advantages, and solutions to problems have been described herein with regard to specific embodiments. Furthermore, the connecting lines shown in the various figures contained herein are intended to represent exemplary functional relationships and/or physical couplings between the various elements. It should be noted that many additional functional relationships or physical connections may be present in a practical system. However, the benefits, advantages, solutions to problems, and any elements that may cause any benefit, advantage, or solution to occur or become more pronounced are not to be construed as critical, required, or essential features or elements of the disclosure.

The scope of the disclosure is accordingly to be limited by nothing other than the appended claims, in which reference to an element in the singular is not intended to mean "one and only one" unless explicitly so stated, but rather "one or more." It is to be understood that unless specifically stated otherwise, references to "a," "an," and/or "the" may include one or more than one and that reference to an item in the singular may also include the item in the plural. All ranges and ratio limits disclosed herein may be combined.

Moreover, where a phrase similar to "at least one of A, B, or C" is used in the claims, it is intended that the phrase be interpreted to mean that A alone may be present in an embodiment, B alone may be present in an embodiment, C alone may be present in an embodiment, or that any combination of the elements A, B and C may be present in a single embodiment; for example, A and B, A and C, B and C, or A and B and C. Different cross-hatching is used throughout the figures to denote different parts but not necessarily to denote the same or different materials.

The steps recited in any of the method or process descriptions may be executed in any order and are not necessarily limited to the order presented. Furthermore, any reference to singular includes plural embodiments, and any reference to more than one component or step may include a singular embodiment or step. Elements and steps in the figures are illustrated for simplicity and clarity and have not necessarily been rendered according to any particular sequence. For example, steps that may be performed concurrently or in different order are illustrated in the figures to help to improve understanding of embodiments of the present disclosure.

Any reference to attached, fixed, connected or the like may include permanent, removable, temporary, partial, full and/or any other possible attachment option. Additionally, any reference to without contact (or similar phrases) may also include reduced contact or minimal contact. Surface shading lines may be used throughout the figures to denote different parts or areas but not necessarily to denote the same or different materials. In some cases, reference coordinates may be specific to each figure.

Systems, methods and apparatus are provided herein. In the detailed description herein, references to "one embodiment", "an embodiment", "various embodiments", etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described.

As used herein, the terms "comprises", "comprising", or any other variation thereof, are intended to cover a non-exclusive inclusion, such that a process, method, article, or apparatus that comprises a list of elements does not include only those elements but may include other elements not expressly listed or inherent to such process, method, article, or apparatus.

## Claims

1. A disinfection system (100) for a cabin of an aircraft, the disinfection system comprising:
a stowable item;
a housing (110) defining a compartment within which the stowable item is configured to be at least partially retained, wherein the housing is a section of a cabin structure of the aircraft;
an electromagnetic radiation source (120) coupled to the housing and configured to operably emit disinfecting electromagnetic radiation to the compartment to deliver a disinfecting treatment to the stowable item;
a controller (130) electrically coupled to the electromagnetic radiation source, the controller comprising a processor and a tangible, non-transitory computer-readable storage medium having instructions stored thereon that, in response to execution by the processor, cause the processor to perform various operations comprising controlling, by the processor, emission of the disinfecting electromagnetic radiation; and
a detector (140) coupled to the housing and electrically coupled to the controller, the detector configured to determine, by the processor, at least one of a presence, a position, and an orientation of the stowable item, wherein controlling the emission of the disinfecting electromagnetic radiation is based on at least one of the presence, the position, and the orientation of the stowable item;
**characterized in that**,
the housing comprises a refractive material configured to direct emission of the disinfecting electromagnetic radiation.

2. The disinfection system of claim 1, wherein the disinfecting electromagnetic radiation configured to be emitted from the electromagnetic radiation source is ultraviolet ("UV") radiation and comprises a wavelength between about 10 nanometers and about 405 nanometers.

3. The disinfection system of claim 1, wherein the disinfecting electromagnetic radiation configured to be emitted from the electromagnetic radiation source is far UV-C radiation and comprises a wavelength between about 100 nanometers and about 280 nanometers.

4. The disinfection system of claim 1, wherein the disinfecting electromagnetic radiation configured to be emitted from the electromagnetic radiation source is UV-B radiation and comprises a wavelength between about 280 nanometers and about 315 nanometers.

5. The disinfection system of claim 1, wherein the disinfecting electromagnetic radiation configured to be emitted from the electromagnetic radiation source is UV-A radiation and comprises a wavelength between about 315 nanometers and about 405 nanometers.

6. The disinfection system of claim 1, wherein controlling the emission of the disinfecting electromagnetic radiation comprises varying a wavelength of the disinfecting electromagnetic radiation.

7. The disinfection system of claim 1, wherein the controller (130) is configured to determine, by the processor, a dosage of the disinfecting electromagnetic radiation delivered to the stowable item.

8. The disinfection system of claim 1, wherein the stowable item is at least one of a personal item of a passenger and a component of the cabin of the aircraft.

9. The disinfection system of claim 8, wherein the housing (110) comprises a passenger storage container and the stowable item comprises the personal item of the passenger, and preferably wherein the passenger storage container comprises a lid, wherein controlling the emission of the disinfecting electromagnetic radiation is based on whether the lid is open or closed.

10. The disinfection system of claim 8, wherein the housing (110) comprises seat structure, the stowable item comprises a tray, and the compartment is a receptacle for at least partially receiving the tray.

11. The disinfection system of claim 10, wherein the seat structure is a seat-back and the electromagnetic radiation source is mounted to the seat-back, and preferably wherein the electromagnetic radiation source is disposed along a shoulder of the seat-back that defines the receptacle.

12. The disinfection system of claim 10, wherein the seat structure is an armrest and the electromagnetic radiation source is disposed within the receptacle.

13. A method of delivering disinfecting electromagnetic radiation to a cabin of an aircraft, the method comprising:
determining, by a processor, at least one of a presence, a position, and an orientation of a stowable item within a housing defining a compartment in the cabin of the aircraft; and
controlling, by the processor, emission of the disinfecting electromagnetic radiation to the compartment to deliver a disinfecting treatment to the stowable item, wherein the housing comprises a refractive material configured to direct emission of the disinfecting electromagnetic radiation, and wherein controlling the emission of the disinfecting electromagnetic radiation is based on at least one of the presence, the position, and the orientation of the stowable item.

## Patentansprüche

1. Desinfizierungssystem (100) für eine Kabine eines Flugzeugs, wobei das Desinfizierungssystem Folgendes umfasst:
einen verstaubaren Artikel;
ein Gehäuse (110), das ein Fach definiert, in dem der verstaubare Artikel konfigurationsgemäß zumindest teilweise aufbewahrt wird, wobei das Gehäuse ein Abschnitt einer Kabinenstruktur des Flugzeugs ist;
eine elektromagnetische Strahlungsquelle (120), die an das Gehäuse gekoppelt und dazu konfiguriert ist, desinfizierende elektromagnetische Strahlung wirksam zu dem Fach zu emittieren, um dem verstaubaren Artikel eine desinfizierende Behandlung bereitzustellen;
eine Steuerung (130), die elektrisch an die elektromagnetische Strahlungsquelle gekoppelt ist, wobei die Steuerung einen Prozessor und ein greifbares, nicht transitorisches computerlesbares Speichermedium umfasst, auf dem Anweisungen gespeichert sind, die als Reaktion auf eine Ausführung durch den Prozessor den Prozessor veranlassen, verschiedene Vorgänge durchzuführen, umfassend Steuern der Emission der desinfizierenden elektromagnetischen Strahlung durch den Prozessor; und
einen Detektor (140), der an das Gehäuse gekoppelt und elektrisch an die Steuerung gekoppelt ist, wobei der Detektor dazu konfiguriert ist, durch den Prozessor mindestens eines von einem Vorhandensein, einer Position und einer Ausrichtung des verstaubaren Artikels zu bestimmen, wobei das Steuern der Emission der desinfizierenden elektromagnetischen Strahlung auf mindestens einem von dem Vorhandensein, der Position und der Ausrichtung des verstaubaren Artikels basiert;
**dadurch gekennzeichnet, dass**
das Gehäuse ein refraktives Material umfasst, das dazu konfiguriert ist, die Emission der desinfizierenden elektromagnetischen Strahlung zu lenken.

2. Desinfizierungssystem nach Anspruch 1, wobei die desinfizierende elektromagnetische Strahlung, die dazu konfiguriert ist, von der elektromagnetischen Strahlungsquelle emittiert zu werden, ultraviolette ("UV") Strahlung ist und eine Wellenlänge zwischen etwa 10 Nanometern und etwa 405 Nanometern umfasst.

3. Desinfizierungssystem nach Anspruch 1, wobei die desinfizierende elektromagnetische Strahlung, die dazu konfiguriert ist, von der elektromagnetischen Strahlungsquelle emittiert zu werden, Fern-UV-C-Strahlung ist und eine Wellenlänge zwischen etwa 100 Nanometern und etwa 280 Nanometern umfasst.

4. Desinfizierungssystem nach Anspruch 1, wobei die desinfizierende elektromagnetische Strahlung, die dazu konfiguriert ist, von der elektromagnetischen Strahlungsquelle emittiert zu werden, UV-B-Strahlung ist und eine Wellenlänge zwischen etwa 280 Nanometern und etwa 315 Nanometern umfasst.

5. Desinfizierungssystem nach Anspruch 1, wobei die desinfizierende elektromagnetische Strahlung, die dazu konfiguriert ist, von der elektromagnetischen Strahlungsquelle emittiert zu werden, UV-A-Strahlung ist und eine Wellenlänge zwischen etwa 315 Nanometern und etwa 405 Nanometern umfasst.

6. Desinfizierungssystem nach Anspruch 1, wobei das Steuern der Emission der desinfizierenden elektromagnetischen Strahlung Variieren einer Wellenlänge der desinfizierenden elektromagnetischen Strahlung umfasst.

7. Desinfizierungssystem nach Anspruch 1, wobei die Steuerung (130) dazu konfiguriert ist, durch den Prozessor eine Dosierung der desinfizierenden elektromagnetischen Strahlung zu bestimmen, die an den verstaubaren Artikel abgegeben wird.

8. Desinfizierungssystem nach Anspruch 1, wobei der verstaubare Artikel mindestens eines von einem persönlichen Artikel eines Passagiers und einer Komponente der Flugzeugkabine ist.

9. Desinfizierungssystem nach Anspruch 8, wobei das Gehäuse (110) einen Aufbewahrungsbehälter für Passagiere umfasst und der verstaubare Artikel den persönlichen Artikel des Passagiers umfasst, und wobei der Aufbewahrungsbehälter für Passagiere vorzugsweise einen Deckel umfasst, wobei das Steuern der Emission der desinfizierenden elektromagnetischen Strahlung darauf basiert, ob der Deckel geöffnet oder geschlossen ist.

10. Desinfizierungssystem nach Anspruch 8, wobei das Gehäuse (110) eine Sitzstruktur umfasst, der verstaubare Artikel eine Ablage umfasst und das Fach eine Aufnahmeeinrichtung zum zumindest teilweisen Aufnehmen der Ablage ist.

11. Desinfizierungssystem nach Anspruch 10, wobei die Sitzstruktur eine Rückenlehne ist und die elektromagnetische Strahlungsquelle an der Rückenlehne montiert ist, und wobei die elektromagnetische Strahlungsquelle vorzugsweise entlang eines Schulterstücks der Rückenlehne angeordnet ist, das die Aufnahmeeinrichtung definiert.

12. Desinfizierungssystem nach Anspruch 10, wobei die Sitzstruktur eine Armlehne ist und die elektromagnetische Strahlungsquelle innerhalb der Aufnahmeeinrichtung angeordnet ist.

13. Verfahren zum Abgeben desinfizierender elektromagnetischer Strahlung an eine Flugzeugkabine, wobei das Verfahren Folgendes umfasst:
Bestimmen mindestens eines von einem Vorhandensein, einer Position und einer Ausrichtung eines verstaubaren Artikels innerhalb eines Gehäuses, das ein Fach in der Kabine des Flugzeugs definiert, durch einen Prozessor; und
Steuern der Emission der desinfizierenden elektromagnetischen Strahlung an das Fach durch den Prozessor, um dem verstaubaren Artikel eine desinfizierende Behandlung bereitzustellen, wobei das Gehäuse ein refraktives Material umfasst, das dazu konfiguriert ist, die Emission der desinfizierenden elektromagnetischen Strahlung zu lenken, und wobei das Steuern der Emission der desinfizierenden elektromagnetischen Strahlung auf mindestens einem von dem Vorhandensein, der Position und der Ausrichtung des verstaubaren Artikels basiert.

## Revendications

1. Système de désinfection (100) pour une cabine d'un aéronef, le système de désinfection comprenant :
un article escamotable ;
un boîtier (110) définissant un compartiment à l'intérieur duquel l'article escamotable est configuré pour être au moins en partie retenu, dans lequel le boîtier est une section d'une structure de cabine de l'aéronef ;
une source de rayonnement électromagnétique (120) couplée au boîtier et configurée pour émettre de manière fonctionnelle un rayonnement électromagnétique désinfectant vers le compartiment pour distribuer un traitement désinfectant à l'article escamotable ;
un contrôleur (130) couplé électriquement à la source de rayonnement électromagnétique, le contrôleur comprenant un processeur et un support de stockage tangible, non transitoire, lisible par ordinateur, sur lequel sont stockées des instructions qui, en réponse à une exécution par le processeur, amènent le processeur à effectuer diverses opérations comprenant le contrôle, par le processeur, de l'émission du rayonnement électromagnétique désinfectant ; et
un détecteur (140) couplé au boîtier et couplé électriquement au contrôleur, le détecteur étant configuré pour déterminer, par le processeur, au moins l'une d'une présence, d'une position et d'une orientation de l'article escamotable, dans lequel le contrôle de l'émission du rayonnement électromagnétique désinfectant est basé sur au moins l'une de la présence, de la position et de l'orientation de l'article escamotable ;
**caractérisé en ce que** le boîtier comprend un matériau réfractif configuré pour diriger l'émission du rayonnement électromagnétique désinfectant.

2. Système de désinfection selon la revendication 1, dans lequel le rayonnement électromagnétique désinfectant configuré pour être émis à partir de la source de rayonnement électromagnétique est un rayonnement ultraviolet (« UV ») et comprend une longueur d'onde comprise entre environ 10 nanomètres et environ 405 nanomètres.

3. Système de désinfection selon la revendication 1, dans lequel le rayonnement électromagnétique désinfectant configuré pour être émis à partir de la source de rayonnement électromagnétique est un rayonnement UV-C lointain et comprend une longueur d'onde comprise entre environ 100 nanomètres et environ 280 nanomètres.

4. Système de désinfection selon la revendication 1, dans lequel le rayonnement électromagnétique désinfectant configuré pour être émis à partir de la source de rayonnement électromagnétique est un rayonnement UV-B et comprend une longueur d'onde comprise entre environ 280 nanomètres et environ 315 nanomètres.

5. Système de désinfection selon la revendication 1, dans lequel le rayonnement électromagnétique désinfectant configuré pour être émis à partir de la source de rayonnement électromagnétique est un rayonnement UV-A et comprend une longueur d'onde comprise entre environ 315 nanomètres et environ 405 nanomètres.

6. Système de désinfection selon la revendication 1, dans lequel le contrôle de l'émission du rayonnement électromagnétique désinfectant comprend la variation d'une longueur d'onde du rayonnement électromagnétique désinfectant.

7. Système de désinfection selon la revendication 1, dans lequel le contrôleur (130) est configuré pour déterminer, par le processeur, un dosage du rayonnement électromagnétique désinfectant distribué à l'article escamotable.

8. Système de désinfection selon la revendication 1, dans lequel l'article escamotable est au moins l'un d'un article personnel d'un passager et d'un composant de la cabine de l'aéronef.

9. Système de désinfection selon la revendication 8, dans lequel le boîtier (110) comprend un contenant de stockage de passager et l'article escamotable comprend l'article personnel du passager, et de préférence dans lequel le contenant de stockage de passager comprend un couvercle, dans lequel le contrôle de l'émission du rayonnement électromagnétique désinfectant est basé sur le fait que le couvercle est ouvert ou fermé.

10. Système de désinfection selon la revendication 8, dans lequel le boîtier (110) comprend une structure de siège, l'article escamotable comprend un plateau, et le compartiment est un réceptacle destiné à recevoir au moins en partie le plateau.

11. Système de désinfection selon la revendication 10, dans lequel la structure de siège est un dossier de siège et la source de rayonnement électromagnétique est montée sur le dossier de siège, et de préférence dans lequel la source de rayonnement électromagnétique est disposée le long d'un épaulement du dossier de siège qui définit le réceptacle.

12. Système de désinfection selon la revendication 10, dans lequel la structure de siège est un accoudoir et la source de rayonnement électromagnétique est disposée à l'intérieur du réceptacle.

13. Procédé de distribution d'un rayonnement électromagnétique désinfectant à une cabine d'un aéronef, le procédé comprenant :
la détermination, par un processeur, d'au moins l'une d'une présence, d'une position et d'une orientation d'un article escamotable dans un boîtier définissant un compartiment dans la cabine de l'aéronef ; et
le contrôle, par le processeur, de l'émission du rayonnement électromagnétique désinfectant vers le compartiment pour distribuer un traitement désinfectant à l'article escamotable, dans lequel le boîtier comprend un matériau réfractif configuré pour diriger l'émission du rayonnement électromagnétique désinfectant, et dans lequel le contrôle de l'émission du rayonnement électromagnétique désinfectant est basé sur au moins l'une de la présence, de la position et de l'orientation de l'article escamotable.
